# EUROPEAN PATENT SPECIFICATION

(11) **EP 3 186 361 B1**
(45) Date of publication and mention of the grant of the patent: **05.06.2019**
(21) Application number: 15778720.1
(22) Date of filing: 31.08.2015
(51) Int. Cl.: C12N 1/20, C12N 7/00, C12R 1/46

(54) **ENTEROCOCCUS FAECALIS STRAINS FOR THE PRODUCTION OF BACTERIOPHAGE PREPARATIONS**
ENTEROCOCCUS FAECALIS STÄMME FÜR DIE HERSTELLUNG VON BAKTERIOPHAGEN
SOUCHES D'ENTEROCOCCUS FAECALIS POUR LA PREPARATION DE BACTERIOPHAGES

(30) Priority: 31.08.2014 PL 40933214
(43) Date of publication of application: 05.07.2017
(60) Divisional application: 18195200.3
(73) Proprietor: Instytut Biochemii I Biofizyki Polskiej Akademii Nauk, 02-106 Warszawa (PL); Instytut Immunologii i Terapii Doswiadczalnej Polskiej Akademii Nauk, 53-114 Wroclaw (PL)
(72) Inventor: LOBOCKA, Malgorzata, PL-02-798 Warszawa (PL); GOZDEK, Agnieszka, PL-00-714 Warszawa (PL); IZAK, Dariusz, PL-05-803 Pruszków (PL); ZALEWSKA, Agnieszka, PL-02-778 Warszawa (PL); GAWOR, Jan, PL-04-357 Warszawa (PL); DABROWSKI, Kamil, PL-05-071 Sulejówek (PL); GROMADKA, Robert, PL-01-960 Warszawa (PL); WEBER-DABROWSKA, Beata, PL-51-628 Wroclaw (PL); GÓRSKI, Andrzej, PL-02-784 Warszawa (PL)
(74) Representative: Witek, Rafal
(86) International application number: PCT/IB2015/056607
(87) International publication number: WO 2016/030872

(56) References cited:
- BOURGOGNE AGATHE ET AL: "Large scale variation in Enterococcus faecalis illustrated by the genome analysis of strain OG1RF", GENOME BIOLOGY, BIOMED CENTRAL LTD., LONDON, GB, vol. 9, no. 7, 8 July 2008 (2008-07-08), page R110, XP021041654, ISSN: 1465-6906
- M. FRITZENWANKER ET AL: "Complete Genome Sequence of the Probiotic Enterococcus faecalis Symbioflor 1 Clone DSM 16431", GENOME ANNOUNCEMENTS, vol. 1, no. 1, 7 February 2013 (2013-02-07), pages e00165-12, XP55233786, DOI: 10.1128/genomeA.00165-12
- MATOS RENATA C ET AL: "Enterococcus faecalis Prophage Dynamics and Contributions to Pathogenic Traits", PLOS GENETICS, vol. 9, no. 6, June 2013 (2013-06), XP055233703,
- FRIEDERIKE S. ROSSMANN ET AL: "Phage-mediated Dispersal of Biofilm and Distribution of Bacterial Virulence Genes Is Induced by Quorum Sensing", PLOS PATHOGENS, vol. 11, no. 2, 23 February 2015 (2015-02-23), page e1004653, XP55233701, DOI: 10.1371/journal.ppat.1004653
- JUMPEI UCHIYAMA ET AL: "In silico and in vivo evaluation of bacteriophage OEF24C, a candidate for treatment of Enterococcusfaecalis infections", APPLIED AND ENVIRONMENTAL MICROBIOLOGY, AMERICAN SOCIETY FOR MICROBIOLOGY, US, vol. 74, no. 13, 1 July 2008 (2008-07-01), pages 4149-4163, XP002633409, ISSN: 0099-2240, DOI: 10.1128/AEM.02371-07 [retrieved on 2008-05-02] cited in the application

## Description

The object of the invention is *Enterococcus faecalis* strain for obtaining monoclonal preparations of lytic bacteriophages with high titer, particularly including preparations of bacteriophages for therapeutic applications. The strain being the result of the invention guarantee the production of safe to use monoclonal therapeutic bacteriophage preparations, not contaminated with additional, undesired bacteriophages that might be a source of uncontrolled propagation of genes for bacterial virulence.

*Enterococcus faecalis* and *Enterococcus faecium* are Gram-positive lactic acid bacteria. As a natural component of the microflora they inhabit mucous membranes, mainly of the human and animal gastrointestinal system. However, in recent years numerous pathogenic strains of these bacteria have appeared. Moreover, they have become one of the major causes of hospital-derived infections, including many infections that are difficult to fight using antibiotics (Arias and Murray, 2012). The difficulties in fighting infections by *E. faecalis* and *E. faecium* are a result of numerous strains of these bacteria acquiring antibiotic resistance, including the resistance to vancomycin considered the last-chance antibiotic.

In this context, what proves to be particularly promising is phage therapy - treating infections with *Enterococcus* strains resistant to antibiotics by using bacteriophages - viruses infecting only bacteria and harmless to cells of eukaryotic organisms (Górski et al., 2009; Golka et al., 2014). The effectiveness thereof in eradicating pathogenic *Enterococcus* strains was demonstrated not only in animal models but also in humans (Uchiyama et al., 2008; Letkiewicz et al., 2009).

Only the so-called obligate lytic bacteriophages are suited for therapeutic applications (Gill and Hyman, 2010). After infecting a bacterium, they may multiply therein only through lytic development pathway, leading to bacterial lysis correlated with its death (Guttman et al., 2005). The bacteriophages excluded from therapeutic applications, referred to as temperate, can, after infecting a bacterium, propagate through the lytic development pathway or remain for many generations in a latent form, namely a prophage, typically being a bacteriophage DNA integrated into bacterial chromosome. In defense against their prophages being lost from bacterial chromosomes, temperate bacteriophages have acquired in the course of evolution a variety of genes beneficial for environmental adaptation of their bacterial hosts. In case of pathogenic bacteria bacteriophages, these are typically genes associated with bacterial virulence, encoding *inter alia* toxins, adhesins or factors protecting the bacteria from immune response (Cheetham et al., 1995). As a result, the acquisition of new prophages is one of the major driving forces for pathogenic bacteria evolution (Canchaya et al., 2003). Prophages are also a problem during bacteriophage production for therapeutic purposes. The latter usually multiply efficiently in bacteria of the same species as the pathogens they fight against (Gill and Hyman, 2010; Balogh et al., 2010). Meanwhile, prophages and fragments thereof are typical elements in genomes of pathogenic bacteria (Canchaya et al., 2003). In some cells of a population carrying an active prophage, a spontaneous excision thereof and initiation of lytic development may occur. There were reports regarding the presence in bacterial cultures of numerous temperate phages, which were released due to spontaneous prophage induction (Wang et al., 2010). These phages, after infecting new hosts may integrate into their chromosomes as prophages, becoming vectors in dissemination of bacterial virulence genes. Therefore, the only guarantee of obtaining monoclonal therapeutic phage preparations, not comprising other phages except the ones used for multiplication, is to employ bacterial strains devoid of prophages to the production thereof. This problem proves to be particularly significant when producing therapeutic bacteriophage preparations against *E. faecalis* and *E. faecium.* Study results from recent years unambiguously indicate the critical role of prophages in the evolution of strains of these bacteria, particularly well adapted to spreading in hospital conditions (Giridhara Upadhyaya et al., 2009; Matos et al., 2013). Very few Enterococcus faecalis strains devoid of prophages are known. For example genome analysis of Enterococcus faecalis strain OG1RF revealed the absence of prophages in this strain (Bourgogne et al. 2008).

The aim of the invention is to provide *Enterococcus faecalis* strains for production of therapeutic bacteriophage preparations with high titer, devoid of contamination with temperate phages. Unexpectedly, the thus defined aim has been achieved due to the solution proposed by the present invention.

The object of the invention is *Enterococcus faecalis* strains devoid of prophages.

The strain according to the invention is the *Enterococcus faecalis* 1679wph strain deposited in the PCM (Polish Collection of Microorganisms) as B/00074.

Preferably, the object of the invention is an *Enterococcus faecalis* strain 1679wph that has a bacterial chromosome comprising DNA with a sequence SEQ ID NO: 1.

The object of the invention is also a method of producing a bacteriophage preparation, particularly a therapeutic one, characterized by the fact that a bacteriophage strain specific against *Enterococcus faecalis* is multiplied in culture of the *Enterococcus faecalis* strain devoid of prophages according to the invention as defined above, and then the multiplied phages are isolated from the obtained lysate, wherein the obtained bacteriophage preparations are devoid of contamination with temperate phages.

In a search for the *Enterococcus faecalis* strains devoid of active prophages, the present inventors screened a collection of strains of this bacterial species for therapeutic bacteriophage multiplication, regarding their sensitivity to mitomycin C - an agent capable of inducing prophage excision and bacterial lysis, associated with lytic development thereof. By analyzing genomic DNA sequences of strains insensitive to mitomycin that were determined for the purposes of the invention, two strains were isolated, not having regions corresponding to active prophages in their chromosomes. Unexpectedly, it was found that the *Enterococcus faecalis* strains not comprising active prophages may be adapted for efficient multiplication of therapeutic bacteriophages against infections caused by the *Enterococcus* strains.

The effect of the invention is the isolation of the 1679wph *E. faecalis* strains, having genomic DNA sequences defined in SEQ ID NO: 1, characterized by the lack of active prophages in their genomes. An advantageous effect of the invention is the possibility to effectively multiply *Enterococcus* bacteriophages in the cells of the 1679wph strain, particularly including bacteriophages comprised in therapeutic preparations. An additional advantageous effect of the invention is the possibility to multiply therapeutic bacteriophages against *Enterococcus* in the 1679wph strain cells, so that the titer of the aforementioned phages comprised in lysates was at least 10⁹ pfu/ml.

For a better understanding of the essence of the invention it is illustrated by the following examples that demonstrate the method of isolation, from a variety of *Enterococcus faecalis* strains for multiplication of therapeutic phages, of strains devoid of active prophages, the method of demonstrating the lack of prophages in genomes of the *E. faecalis* 1679wph and 1854wph strains, and the method of demonstrating that the obtained strains are suitable for use as host strains for efficient multiplication of therapeutic bacteriophages. In all examples, unless stated otherwise, standard microbiology and molecular biology methods were employed, as described by Miller (1972) and Sambrook et al. (1989).

### Example 1. Preliminary analysis of genomic sequence of selected Enterococcus faecalis strains regarding the presence of sequences representing active prophages.

Several *Enterococcus faecalis* strains for multiplication of therapeutic bacteriophages, from the collection of The Ludwik Hirszfeld Institute of Immunology and Experimental Therapy, Polish Academy of Sciences, were purified through single colonies in order to obtain homogeneous clones, and then cells from these colonies were seeded on fresh LB medium (2% triton, Difco; 0.5% yeast extract, Difco; 0.5% NaCl; pH 7.5). Cells suspended in medium were treated with mitomycin C and different dilutions of thus treated suspensions were seeded on plates with complete medium. In case of four strains, no difference was observed in the number of colonies growing on plates after seeding mitomycin C treated cells, in comparison to control cells, which might indicate the lack of active prophages in chromosomal DNA of these strains. Isolated DNA of these strains was used to prepare libraries of fragments that were subsequently subjected to sequencing, using the Roche 454 Genome Sequencer GS FLX, according to previously described methodology ( 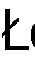 obocka et al., 2012). Analysis of the preassembled sequence fragments to identify DNA regions exhibiting features of active prophages, allowed selecting two strains as potentially lacking such regions. Accordingly, larger libraries of DNA fragments from these strains were prepared and sequenced using the MiSeq Small Genome Sequence utilizing the Illumina technology (Illumina). Assembling the obtained data allowed to obtain almost complete drafts of genomic sequences for the identified strains, designated for the purposes of the present invention as 1679wph (SEQ ID NO: 1) (2 853 265 base pairs) and 1854wph (SEQ ID NO: 2) (2 858 082 base pairs). Small fragments with an undetermined order of nucleotide residues in the draft sequences (one fragment about 150 residues long in the 1679wph strain sequence and 6 fragments about 2000, 25, 1700, 2240, 4800 and 960 residues long) were several times too short to be able to correspond to integrated prophage sequences. On the other hand, a thorough analysis of the obtained genomic sequences for both strains, both by searching for homology with DNA sequences in the GenBank database, as well as by searching for homology at the protein level after translating the obtained sequences to protein sequences for all reading frames, confirmed the preliminary results indicating the lack of fragments in their genomes that might represent active prophage genomes. Additionally, both sequences shown 99% identity indicating close relationship of both strains.

The obtained *Enterococcus faecalis* strains devoid of prophages and plasmids were deposited in the Polish Collection of Microorganisms (PCM), Wroclaw, Poland, according to the Budapest Treaty.

| *Enterococcus faecalis* strain designation | Polish Collection of Microorganisms (PCM) deposit accession number |
|---|---|
| 1679wph | B/00074 |
| 1854wph | B/00075 |

### Example 2. Differentiating the 1679wph and 1854wph strains in terms of sensitivity to Enterococcus phages.

In order to test whether, despite their high similarity at the level of DNA sequence, the 1679wph and 1854wph strains differ in terms of sensitivity to phages, the susceptibility of these strains to infection with different phages comprised in thirteen different therapeutic phage preparations against *Enterococcus* derived from the collection of the Bacteriophage Laboratory of the Ludwik Hirszfeld Institute of Immunology and Experimental Therapy, Polish Academy of Sciences (Table 1). Lysates with titer of 10⁸-10⁹, obtained as a result of phage multiplication in cells of various *Enterococcus* strains, previously selected as suitable for efficient multiplication of these phages and derived from the collection of the Bacteriophage Laboratory of the Institute of Immunology and Experimental Therapy, Polish Academy of Sciences. Phages were multiplied by adding a phage suspension to a liquid culture of an *Enterococcus faecalis* strain in an exponential growth phase (with optical density of OD600 = ∼0.35) in LB medium, so that the multiplicity of infection (M.O.I.) was 1. Additionally, MgSO₄ and CaCl₂ were added to the culture (to a final concentration of 10 mM). The mixture was allowed to stand for 10 min. at room temperature and then was incubated at 37°C for about 90 minutes with shaking. If lysis did not occur, incubation was extended overnight. The lysate was centrifuged (20 min., 4°C, 9000 g), and the obtained supernatant was filtered through 0.22 PVDF filters (from MILLEX® GS). The infectivity assay was conducted by the double-layer plates method on LB medium, as previously described (Golec et al., 2011). It was unexpectedly found that the cells of the 1679wph strain are infected by all the tested phages and the cells of the 1854wph strain by 12 of the 13 tested phages. Furthermore, small differences were observed in the infection efficiency for the cells of the 1679wph and 1854wph strains by some phages, suggesting that the 1679wph and 1854wph strains, despite their similarity at the level of genomic DNA sequence, differ in their sensitivity to specific phages.

### Example 3. Verification of the 1679wph strain usefulness as a universal host for efficient multiplication of Enterococcus faecalis bacteriophages.

In relation to the infectivity of many different *Enterococcus* bacteriophages against the cells of closely related 1979wph and 1854wph strains, it was examined, in the case of 1679wph, whether following the adaptation of bacteriophages, previously multiplied in cells of other *Enterococcus* strains to multiplication in the cells of the 1679wph strain, the 1679wph strain could serve as a host for efficient multiplication of these bacteriophages. Individual plaques, formed in 1679wph strain cell layer by the phages referred in Table 1, were isolated and the phages contained therein were used to infect cells of the 1979wph strain. Then, bacteriophages obtained by multiplication in 1679wph cells were used again to infect 1679wph cells for subsequent phage multiplication, as described in example 1. Following the observation of cell lysis symptoms the culture was discontinued and the lysate was purified of cell debris, as described in example 1. The titer of phages comprised in the thus obtained lysates was determined in the 1679wph strain cell layer, according to the description in example 1. It was unexpectedly found that the titers of all tested bacteriophages in lysates obtained from cells of the 1679wph strain were at least 10⁹, which meets the standards expected for therapeutic phage preparations (Merabishvili et al., 2009). It is therefore clear that the E. *faecalis* 1679wph strain, as well as the 1854wph strain closely related thereto, are both suitable hosts for obtaining therapeutic bacteriophage preparations of high titer of phage particles.

**Table 1. Infectivity* of Enterococcus bacteriophages multiplied in cells of various Enterococcus strains, against E. faecalis 1679wph and 1854wph strains.**

| Phage preparation | Enterococcus strain used for multiplication | Number of plaques (given as pfu/ml) on the cell layer of Enterococcus faecalis strain: | |
|---|---|---|---|
| | | **1679wph** | **1854wph** |
| EF1 | KRZ | 8 x 10⁶ | 6 x 10⁶ |
| EF3 | KRZ | 2 x 10⁷ | 1 x 10⁷ |
| EF12 | KRZ | 6 x 10⁶ | 8 x 10⁶ |
| EF13 | ADA | 2 x 10⁶ | 4 x 10⁷ |
| EF15 | PIE | 2 x 10⁵ | 2 x 10⁷ |
| EF49 | 1679 | 1 x 10⁸ | 2 x 10⁸ |
| EF56 | 1854 | 6 x 10⁸ | 5 x 10⁸ |
| EF4 | RYB | 1 x 10⁹ | 1 x 10⁹ |
| EF7 | PIE | 2 x 10⁷ | 2 x 10⁷ |
| EF37 | 1515 | 3 x 10⁹ | 3 x 10⁹ |
| EF39 | 27677 | 2 x 10⁹ | 2 x 10⁹ |
| EF57 | 1854 | 3 x 10⁹ | 6 x 10⁸ |
| EF62 | 2451 | 8 x 10⁴ | Lack of plaques |

| | | | |
|---|---|---|---|
| * Plaque number determined on the basis of count thereof on plates with cell layer of the particular strain is expressed per ml of lysate used for infection (pfu/ml). | | | |

**Table 2. Enterococcus bacteriophage titer in lysates obtained after adaptation and multiplication in cells of the E. faecalis 1679wph strain.**

| **Lysates** | **Enterococcus strain of the previous multiplication host** | **Phage titer in lysates multiplied in cells of the *E*. *faecalis* 1679wph strain, after adaptation** (pfu/ml) |
|---|---|---|
| EF1 | KRZ | 6 x 10⁹ |
| EF3 | KRZ | 1 x 10⁹ |
| EF12 | KRZ | 1 x 10⁹ |
| EF13 | ADA | 7 x 10⁹ |
| EF15 | PIE | 1 x 10⁹ |
| EF49 | 1679 | 5 x 10¹⁰ |
| EF56 | 1854 | 1 x 10¹⁰ |
| EF4 | RYB | 9 x 10⁹ |
| EF7 | PIE | 7 x 10⁹ |
| EF37 | 1515 | 1 x 10¹⁰ |
| EF39 | 27677 | 9 x 10⁹ |
| EF57 | 1854 | 4 x 10¹⁰ |
| EF62 | 2451 | 4 x 10⁸ |
| EF24 | 661 | 1 x 10⁹ |

### Literature

Arias CA, Murray BE. 2012. The rise of the Enterococcus: beyond vancomycin resistance. Nat Rev Microbiol. 10: 266-278.
Balogh, B.; Jones, J. B.; Iriarte, F. B.; Momol, M. T. Phage therapy for plant disease control. Curr. Pharm. Biotechnol., 2010, 11: 48-57.
Bourgogne A. et al. (2008). Large scale variation in Enterococcus faecalis illustrated by the genome analysis of strain OG1RF. Genome Biology 9: R110.
Canchaya C, Proux C, Fournous G, Bruttin A, Brüssow H. 2003. Prophage genomics. Microbiol. Mol. Biol. Rev. 67: 238-276,
Cheetham BF, Katz ME. 1995. A role for bacteriophages in the evolution and transfer of bacterial virulence determinants. Mol Microbiol. 18: 201-208.
Gill, J.J., and Hyman, P. 2010. Phage choice, isolation, and preparation for phage therapy. Curr Pharm Biotechnol. 11: 2-14.
Golec, P., D 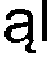 browski, K., Hejnowicz, M.S., Gozdek, A., Loś, J.M., W 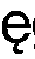 grzyn, G., Lobocka, M.B., and Loś, M. (2011). A reliable method for storage of tailed phages. J Microbiol Methods. 84, 486-9.
Golkar Z, Bagasra O, Pace DG. 2014. Bacteriophage therapy: a potential solution for the antibiotic resistance crisis. J Infect Dev Ctries. 8: 129-136.
Górski A, Miedzybrodzki R, Borysowski J, Weber-Dabrowska B, Lobocka M, Fortuna W, Letkiewicz S, Zimecki M, Filby G. 2009. Bacteriophage therapy for the treatment of infections. Curr Opin Investig Drugs. 10: 766-774.
Giridhara Upadhyaya PM, Ravikumar KL, Umapathy BL. 2009. Review of virulence factors of enterococcus: an emerging nosocomial pathogen. Indian J Med Microbiol. 27: 301-305.
Guttman, B., Raya, P., and Kutter, E. 2005. Basic phage biology. In Bacteriophages: biology and application, E.B., Kutter, and A., Sulakvelidze, eds. (Boca Raton (FL): CRC Press), pp. 29-66.
Letkiewicz S, Miedzybrodzki R, Fortuna W, Weber-Dabrowska B, Górski A. 2009. Eradication of Enterococcus faecalis by phage therapy in chronic bacterial prostatitiscase report. Folia Microbiol (Praha). 54: 457-461.
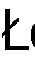 obocka M., Hejnowicz M. S., G 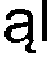 ga 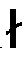 a U., Weber-D 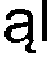 browska B., W 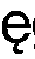 grzyn G., Dadlez M. 2014. The first step to bacteriophage therapy - how to choose the correct phage. In.: Phage Therapy. Current Research and Applications. Borysowski J., Mi 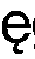 dzybrodzki R., Górski, A. (ed.). Caister Academic Press. pp. 23-69.
Matos RC, Lapaque N, Rigottier-Gois L, Debarbieux L, Meylheuc T, Gonzalez-Zorn B, Repoila F, Lopes Mde F, Serror P. 2013. Enterococcus faecalis prophage dynamics and contributions to pathogenic traits. PLoS Genet. 9(6):e1003539.
Merabishvili, M., Pirnay, J.P., Verbeken, G., Chanishvili, N., Tediashvili, M., Lashkhi, N., Glonti, T., Krylov, V., Mast, J., Van Parys, L., Lavigne, R., Volckaert, G., Mattheus, W., Verween, G., De Corte, P., Rose, T., Jennes, S,, Zizi, M., De Vos, D., and Vaneechoutte, M. 2009. Quality-controlled small-scale production of a well-defined bacteriophage cocktail for use in human clinical trials. PLoS One. 4, e4944.
Miller J.H. 1972. Experiments in molecular genetics. Cold Spring Harbour Laboratory, Cold Spring Harbour. New York.
Uchiyama J, Rashel M, Takemura I, Wakiguchi H, Matsuzaki S. In silico and in vivo evaluation of bacteriophage phiEF24C, a candidate for treatment of Enterococcus faecalis infections. 2008. Appl Environ Microbiol. 74: 4149-4163.
Wang, X., Kim, Y., Ma, Q., Hong, S.H., Pokusaeva, K., Sturino, J.M., and Wood, T.K. 2010. Cryptic prophages help bacteria cope with adverse environments. Nature Communications 1, 147.

## Claims

1. An *Enterococcus faecalis* strain devoid of prophages, **characterized in that** it is the *Enterococcus faecalis* 1679wph strain deposited in the PCM as B/00074.

2. *Enterococcus faecalis* strain according to claim 1 **characterized in that** it has a bacterial chromosome comprising DNA with a sequence SEQ ID NO: 1.

3. A method of producing a bacteriophage preparation, particularly a therapeutic one, **characterized in that** a bacteriophage strain specific against *Enterococcus faecalis* is multiplied in culture of the *Enterococcus faecalis* strain devoid of prophages according to claims 1 - 2, and then the multiplied phages are isolated from the obtained lysate, wherein the obtained bacteriophage preparations are devoid of contamination with temperate phages.

## Patentansprüche

1. *Enterococcus faecalis* Stamm frei von Prophagen, **dadurch gekennzeichnet, dass** er der Stamm *Enterococcus faecalis* 1679wph wie in der PCM als B/00074 hinterlegt ist.

2. *Enterococcus faecalis* Stamm nach Anspruch 1, **dadurch gekennzeichnet, dass** er ein bakterielles Chromosom umfassend eine DNA mit einer Sequenz SEQ ID NO: 1 aufweist.

3. Verfahren zur Herstellung einer Bakteriophagenpräparation, insbesondere einer therapeutischen Bakteriophagenpräparation, **dadurch gekennzeichnet, dass** ein gegen *Enterococcus faecalis* spezifischer Bakteriophagenstamm in einer Kultur des *Enterococcus faecalis* Stammes frei von Prophagen nach den Ansprüchen 1 - 2 vervielfältigt wird, und dann die vervielfältigten Phagen von dem erhaltenen Lysat isoliert werden, wobei die erhaltenen Bakteriophagenpräparationen frei von einer Kontamination mit temperenten Phagen sind.

## Revendications

1. Souche d'*Enterococcus faecalis* dépourvue de prophages, **caractérisée en ce qu'**il s'agit de la souche d'*Enterococcus faecalis* 1679wph déposée auprès de la CPM en tant que B/00074.

2. Souche d'*Enterococcus faecalis* selon la revendication 1, **caractérisée en ce qu'**elle a un chromosome bactérien comprenant un ADN avec une séquence SEQ ID N° : 1.

3. Procédé de production d'une préparation de bactériophages, en particulier une préparation thérapeutique, **caractérisée en ce qu'**une souche bactériophage spécifique dirigée contre *Enterococcus faecalis* est multipliée lors de la culture de la souche *d'Enterococcus faecalis* dépourvue de prophages selon les revendications 1-2, puis les phages multipliés sont isolés du lysat obtenu, dans lequel les préparations de bactériophages obtenues sont dépourvues de contamination avec des phages tempérés.
